# EUROPEAN PATENT SPECIFICATION

(11) **EP 3 291 736 B1**
(45) Date of publication and mention of the grant of the patent: **19.06.2024**
(21) Application number: 16787216.7
(22) Date of filing: 29.04.2016
(51) Int. Cl.: A61B 34/20, A61B 18/18, A61B 8/14, A61B 8/08, A61B 8/00

(54) **MICROWAVE ABLATION PLANNING AND PROCEDURE SYSTEMS**
PLANUNGS- UND VERFAHRENSSYSTEME FÜR MIKROWELLENABLATION
SYSTÈMES DE PLANIFICATION ET DE PROCÉDURE D'ABLATION PAR MICRO-ONDES

(30) Priority: 30.04.2015 US 201562154929 P; 30.04.2015 US 201562154924 P; 30.04.2015 US 201562154933 P; 30.04.2015 US 201562154942 P; 30.04.2015 US 201562154950 P; 15.04.2016 US 201615099730; 15.04.2016 US 201615099665; 15.04.2016 US 201615099772; 15.04.2016 US 201615099698; 15.04.2016 US 201615099820
(43) Date of publication of application: 14.03.2018
(73) Proprietor: Covidien LP, Mansfield, MA 02048 (US)
(72) Inventor: GIROTTO, Darren G., Louisville, Colorado 80027 (US); BHARADWAJ, Jeetendra S., Lafayette, Colorado 80026 (US); FRANK, Kevin J., Louisville, CO 80027 (US)
(74) Representative: Maschio & Soames IP Ltd
(86) International application number: PCT/US2016/030028
(87) International publication number: WO 2016/176549

(56) References cited:
- WO-A2-2010/096419
- JP-A- 2001 340 350
- US-A1- 2002 049 375
- US-A1- 2005 090 742
- US-A1- 2005 090 742
- US-A1- 2010 240 997

## Description

### BACKGROUND

### 1. Technical Field

The present disclosure relates to systems, methods, and devices for planning and performing a microwave ablation treatment procedure.

### 2. Discussion of Related Art

When planning a treatment procedure, clinicians often rely on patient data including X-ray data, computed tomography (CT) scan data, magnetic resonance imaging (MRI) data, or other imaging data that allows the clinician to view the internal anatomy of a patient. The clinician utilizes the patient data to identify targets of interest and to develop strategies for accessing the targets of interest for the surgical procedure.

The use of CT images as a diagnostic tool has become routine and CT results are frequently the primary source of information available to a clinician regarding the size and location of a lesion, tumor or other similar target of interest. This information is used by the clinician for planning an operative procedure such as a biopsy or an ablation procedure, but is only available as "offline" information which must typically be memorized to the best of the clinician's ability prior to beginning a procedure. During a CT scan, a patient is digitally imaged and a CT image data volume is assembled. The CT image data may then be viewed by the clinician each of the axial, coronal and sagittal directions. A clinician reviews the CT image data slice by slice from each direction when attempting to identify or locate a target. It is often difficult, however, for the clinician to effectively plan a surgical ablation procedure based on the X-rays, CT images, or MRIs in their raw form.

WO 2010/096419 discloses methods, systems, devices, and computer-readable media for image guided surgery. The systems herein allow a physician to use multiple instruments for a surgery and simultaneously provide image-guidance data for those instruments. Various examples provide information to physicians about procedures they are performing, the devices (such as ablation needles, ultrasound wands or probes, scalpels, cauterizers, etc.) they are using during the procedure, the relative emplacements or poses of these devices, prediction information for those devices, and other information.

US 2005/090742 ultrasonic diagnostic equipment which is equipped with an ultrasonic probe that transmits/receives ultrasound to/from an examined body, a probe position sensor that detects the position and the direction of the ultrasonic probe, an image generator that generates image data based upon the output of the ultrasonic probe, a probe position sensor that detects the position and the direction of a puncture probe inserted into the examined body, a display image generator that generates the data of a display image in which the end position of the puncture probe is fixed to a specific position in an image display area according to the position and the direction of the ultrasonic probe and the position and the direction of the puncture probe based upon the image data, and a display for displaying the display image in the image display area.

### SUMMARY OF THE INVENTION

The invention is defined by independent claims 1 and 9. Further advantageous embodiments are defined in the dependent claims.

### SUMMARY OF THE DISCLOSURE

Systems, devices, and methods for planning and performing a microwave ablation treatment procedure are provided. According to an aspect of the present disclosure, a system for performing a microwave ablation procedure comprises an ablation probe, an electromagnetic tracking system configured to track the location of the ablation probe inside a patient's body by using at least one electromagnetic sensor located on the ablation probe while the ablation probe is navigated inside the patient's body, an ultrasound imager configured to generate real-time ultrasound images, and a computing device including a processor and a memory storing instructions which, when executed by the processor, cause the computing device to, display guidance for navigating the ablation probe to at least one ablation target within the patient's body, display the tracked location of the ablation probe on the real-time ultrasound images based on the tracked location of the ablation probe inside the patient's body, iteratively update the displayed location of the ablation probe as the location of the ablation probe is tracked while the ablation probe is navigated inside the patient's body, and display guidance for ablating the at least one target when the ablation probe is navigated proximate to the at least one target.

In a further aspect of the present disclosure, the electromagnetic tracking system tracks the location of the ultrasound imager by using at least one electromagnetic sensor located on the ultrasound imager.

In another aspect of the present disclosure, the location of the ablation probe is displayed in relation to the at least one target.

In a further aspect of the present disclosure, the location of the ablation probe in relation to the at least one target is displayed on the real-time ultrasound images based on the tracked location of the ablation probe inside the patient's body.

In another aspect of the present disclosure, the location of the ablation probe in relation to the at least one target is displayed on the real-time ultrasound images based on the tracked location of the ultrasound imager.

In a further aspect of the present disclosure, the displayed location of the ablation probe is iteratively updated in relation to the at least one target.

In another aspect of the present disclosure, the instructions further configure the computing device to display a model of a planned ablation zone in relation to the at least one target on the real-time ultrasound images.

In a further aspect of the present disclosure, the system further comprises one or more peripheral components which are automatically started by the computing device, wherein the one or more peripheral components include one or more of an ablation generator, a peristaltic pump, and a smart power supply.

In another aspect of the present disclosure, the ablation probe is percutaneously inserted into the patient's body.

In a further aspect of the present disclosure, the instructions further configure the computing device to display, on the real-time ultrasound images, a vector from the tip of the ablation probe indicating the trajectory of the ablation probe.

In another aspect of the present disclosure, the instructions further configure the computing device to display, on the real-time ultrasound images, a projected ablation zone relative to the ablation probe.

In a further aspect of the present disclosure, the instructions further configure the computing device to display, on the real-time ultrasound images, a shadow bar overlay indicating whether the trajectory of the ablation probe is in front of or behind the plane of the real-time ultrasound images.

In another aspect of the present disclosure, the computing device enables a user to preconfigure at least one parameter regarding ablating the at least one target.

In a further aspect of the present disclosure, the at least one parameter includes one or more of a wattage, temperature, and duration.

According to another aspect of the present disclosure, a method comprises displaying guidance for navigating an ablation probe to at least one ablation target within a patient's body, tracking the location of the ablation probe inside the patient's body while the ablation probe is navigated, displaying the tracked location of the ablation probe on real-time ultrasound images, iteratively updating the displayed location of the ablation probe as the location of the ablation probe is tracked while the ablation probe is navigated inside the patient's body, and ablating the at least one target when the ablation probe is navigated proximate to the at least one target.

In a further aspect of the present disclosure, the method further comprises tracking the location of at least one electromagnetic sensor located on the ablation probe.

In a another aspect of the present disclosure, the location of the ablation probe is displayed in relation to the at least one target.

In a further aspect of the present disclosure, the location of the ablation probe in relation to the at least one target is displayed on the real-time ultrasound images based on the tracked location of the ablation probe inside the patient's body.

In another aspect of the present disclosure, the method further comprises tracking the location of an ultrasound imager.

In a further aspect of the present disclosure, the location of the ablation probe in relation to the at least one target is displayed on the real-time ultrasound images based on the tracked location of the ultrasound imager.

In yet a further aspect of the present disclosure, the displayed location of the ablation probe is iteratively updated in relation to the at least one target.

In another aspect of the present disclosure, the method further comprises displaying a model of a planned ablation zone in relation to the at least one target on the real-time ultrasound images.

In a further aspect of the present disclosure, the method further comprises automatically starting one or more peripheral components, wherein the one or more peripheral components include one or more of an ablation generator, a peristaltic pump, and a smart power supply.

In another aspect of the present disclosure, the method further comprises inserting the ablation probe percutaneously into the patient's body.

In a further aspect of the present disclosure, the method further comprises displaying, on the real-time ultrasound images, a vector from the tip of the ablation probe indicating the trajectory of the ablation probe.

In another aspect of the present disclosure, the method further comprises displaying, on the real-time ultrasound images, a projected ablation zone relative to the ablation probe.

In a further aspect of the present disclosure, the method further comprises displaying, on the real-time ultrasound images, a shadow bar overlay indicating whether the trajectory of the ablation probe is in front of or behind the plane of the real-time ultrasound images.

In a further aspect of the present disclosure, the method further comprises preconfiguring at least one parameter regarding ablating the at least one target.

In another aspect of the present disclosure, the at least one parameter includes one or more of a wattage, temperature, and duration.

According to another aspect of the present disclosure, a non-transitory computer-readable storage medium stores instructions which, when executed by a processor, cause a computing device to display guidance for navigating an ablation probe to at least one ablation target within a patient's body, track the location of the ablation probe inside the patient's body while the ablation probe is navigated, display the tracked of the ablation probe on real-time ultrasound images, iteratively update the displayed location of the ablation probe as the location of the ablation probe is tracked while the ablation probe is navigated inside the patient's body, and display guidance for ablating the at least one target when the ablation probe is navigated proximate to the at least one target.

In a further aspect of the present disclosure, the computer-readable medium stores further instructions which configure the computing device to track the location of at least one electromagnetic sensor located on the ablation probe.

In another aspect of the present disclosure, the location of the ablation probe is displayed in relation to the at least one target.

In a further aspect of the present disclosure, the location of the ablation probe in relation to the at least one target is displayed on the real-time ultrasound images based on the tracked location of the ablation probe inside the patient's body.

In another aspect of the present disclosure, the computer-readable medium stores further instructions which configure the computing device to track the location of an ultrasound imager.

In a further aspect of the present disclosure, the location of the ablation probe in relation to the at least one target is displayed on the real-time ultrasound images based on the tracked location of the ultrasound imager.

In another aspect of the present disclosure, the displayed location of the ablation probe is iteratively updated in relation to the at least one target.

In a further aspect of the present disclosure, the computer-readable medium stores further instructions which configure the computing device to display a model of a planned ablation zone in relation to the at least one target on the real-time ultrasound images.

In another aspect of the present disclosure, the computer-readable medium stores further instructions which configure the computing device to automatically start one or more peripheral components, wherein the one or more peripheral components include one or more of an ablation generator, a peristaltic pump, and a smart power supply.

In a further aspect of the present disclosure, the ablation probe is inserting percutaneously into the patient's body.

In another aspect of the present disclosure, the computer-readable medium stores further instructions which configure the computing device to display, on the real-time ultrasound images, a vector from the tip of the ablation probe indicating the trajectory of the ablation probe.

In a further aspect of the present disclosure, the computer-readable medium stores further instructions which configure the computing device to display, on the real-time ultrasound images, a projected ablation zone relative to the ablation probe.

In another aspect of the present disclosure, the computer-readable medium stores further instructions which configure the computing device to display, on the real-time ultrasound images, a shadow bar overlay indicating whether the trajectory of the ablation probe is in front of or behind the plane of the real-time ultrasound images.

In a further aspect of the present disclosure, the computer-readable medium stores further instructions which configure the computing device to enable a user to preconfigure at least one parameter regarding ablating the at least one target.

In another aspect of the present disclosure, the at least one parameter includes one or more of a wattage, temperature, and duration.

According to another aspect of the present disclosure, a system for performing a microwave ablation procedure comprising an ablation probe, an electromagnetic tracking system configured to track the location of the ablation probe inside a patient's body by using at least one electromagnetic sensor located on the ablation probe while the ablation probe is navigated inside the patient's body, a computing device including a processor and a memory storing instructions which, when executed by the processor, cause the computing device to display a three-dimensional model of at least a part of a patient's body generated based on image data acquired during imaging of the patient's body,. display a pathway for navigating an ablation probe to at least one ablation target within the patient's body, track the location of the ablation probe inside the patient's body while the ablation probe is navigated along the pathway, display the tracked location of the ablation probe on the three-dimensional model, iteratively update the displayed location of the ablation probe as the location of the ablation probe is tracked while the ablation probe is navigated inside the patient's body, and display guidance for ablating the at least one target when the ablation probe is navigated proximate to the at least one target.

In a further aspect of the present disclosure, the pathway to the at least one target is a straight line.

In another aspect of the present disclosure, the pathway extends between the at least one target and the exterior of the body of the patient.

In a further aspect of the present disclosure, the system further comprises an ultrasound imager configured to generate real-time ultrasound images of the patient's body.

In another aspect of the present disclosure, the electromagnetic tracking system is further configured to track the location of the ultrasound imager using at least one electromagnetic sensor located on the ultrasound imager.

In a further aspect of the present disclosure, the instructions further configure the computing device to display the tracked location of the ablation probe on real-time ultrasound images generated by the ultrasound imager.

In another aspect of the present disclosure, the location of the ablation probe in relation to the at least one target is displayed on the real-time ultrasound images based on the tracked location of the ablation probe inside the patient's body.

In a further aspect of the present disclosure, the location of the ablation probe in relation to the at least one target is displayed on the real-time ultrasound images based on the tracked location of the ultrasound imager.

In another aspect of the present disclosure, the instructions further configure the computing device to display a model of a planned ablation zone in relation to the at least one target on the three-dimensional model.

In a further aspect of the present disclosure, the instructions further configure the computing device to display a model of a planned ablation zone in relation to the at least one target on the real-time ultrasound images.

In another aspect of the present disclosure, the instructions further configure the computing device to display, on the real-time ultrasound images, a projected ablation zone relative to the ablation probe.

In a further aspect of the present disclosure, the instructions further configure the computing device to display, on the three-dimensional model, a projected ablation zone relative to the ablation probe.

In another aspect of the present disclosure, the displayed location of the ablation probe is iteratively updated in relation to the pathway as the location of the ablation probe is tracked while the ablation probe is navigated inside the patient's body.

In a further aspect of the present disclosure, the instructions further configure the computing device to display, on the three-dimensional model, a vector from the tip of the ablation probe indicating the trajectory of the ablation probe.

In another aspect of the present disclosure, the instructions further configure the computing device to display, on the real-time ultrasound images, a vector from the tip of the ablation probe indicating the trajectory of the ablation probe.

In a further aspect of the present disclosure, the instructions further configure the computing device to display, on the real-time ultrasound images, a shadow bar overlay indicating whether the trajectory of the ablation probe is in front of or behind the plane of the real-time ultrasound images.

In another aspect of the present disclosure, the ablation probe is percutaneously inserted into the patient's body.

According to another aspect of the present disclosure, a method comprises displaying a three-dimensional model of at least a part of a patient's body generated based on image data acquired during imaging of the patient's body, displaying a pathway for navigating an ablation probe to at least one ablation target within the patient's body, tracking the location of the ablation probe inside the patient's body while the ablation probe is navigated along the pathway, displaying the tracked location of the ablation probe on the three-dimensional model, iteratively updating the displayed location of the ablation probe as the location of the ablation probe is tracked while the ablation probe is navigated inside the patient's body, and ablating the at least one target when the ablation probe is navigated proximate to the at least one target.

In a further aspect of the present disclosure, the pathway to the at least one target is a straight line.

In another aspect of the present disclosure, the pathway extends between the at least one target and the exterior of the body of the patient.

In a further aspect of the present disclosure, the method further comprises tracking the location of at least one electromagnetic sensor located on the ablation probe.

In another aspect of the present disclosure, the method further comprises tracking the location of an ultrasound imager.

In another aspect of the present disclosure, the method further comprises displaying the tracked location of the ablation probe on real-time ultrasound images generated by the ultrasound imager.

In a further aspect of the present disclosure, the location of the ablation probe in relation to the at least one target is displayed on the real-time ultrasound images based on the tracked location of the ablation probe inside the patient's body.

In another aspect of the present disclosure, the location of the ablation probe in relation to the at least one target is displayed on the real-time ultrasound images based on the tracked location of the ultrasound imager.

In a further aspect of the present disclosure, the method further comprises displaying a model of a planned ablation zone in relation to the at least one target on the three-dimensional model.

In another aspect of the present disclosure, the method further comprises displaying a model of a planned ablation zone in relation to the at least one target on the real-time ultrasound images.

In a further aspect of the present disclosure, the method further comprises displaying, on the real-time ultrasound images, a projected ablation zone relative to the ablation probe.

In another aspect of the present disclosure, the method further comprises displaying, on the three-dimensional model, a projected ablation zone relative to the ablation probe.

In a further aspect of the present disclosure, the displayed location of the ablation probe is iteratively updated in relation to the pathway as the location of the ablation probe is tracked while the ablation probe is navigated inside the patient's body.

In another aspect of the present disclosure, the method further comprises displaying, on the three-dimensional model, a vector from the tip of the ablation probe indicating the trajectory of the ablation probe.

In a further aspect of the present disclosure, the method further comprises displaying, on the real-time ultrasound images, a vector from the tip of the ablation probe indicating the trajectory of the ablation probe.

In another aspect of the present disclosure, the method further comprises displaying, on the real-time ultrasound images, a shadow bar overlay indicating whether the trajectory of the ablation probe is in front of or behind the plane of the real-time ultrasound images.

In a further aspect of the present disclosure, the method further comprises inserting the ablation probe percutaneously into the patient's body.

Any of the above aspects and embodiments of the present disclosure may be combined without departing from the scope of the present disclosure.

### BRIEF DESCRIPTION OF THE DRAWINGS

Objects and features of the presently disclosed system and method will become apparent to those of ordinary skill in the art when descriptions of various embodiments thereof are read with reference to the accompanying drawings, of which:
Fig. 1 is a schematic diagram of a microwave ablation planning and procedure system in accordance with an illustrative embodiment of the present disclosure;
Fig. 2 is a schematic diagram of a computing device which forms part of the microwave ablation planning and procedure system of Fig. 1 in accordance with an embodiment of the present disclosure;
Fig. 3 is flow chart illustrating an example method of a procedure phase of a microwave ablation treatment in accordance with an embodiment of the present disclosure;
Fig. 4 is an illustration of a user interface presenting a view showing a setup step of the procedure phase of the microwave ablation treatment in accordance with an embodiment of the present disclosure,
Fig. 5 is an illustration of a user interface presenting a view showing a guidance step of the procedure phase of the microwave ablation treatment in accordance with an embodiment of the present disclosure;
Fig. 6 is an illustration of a user interface presenting a view showing guidance during the procedure phase of the microwave ablation treatment in accordance with an embodiment of the present disclosure; and
Fig. 7 is an illustration of a user interface presenting a view showing an ablation step of the procedure phase of the microwave ablation treatment in accordance with an embodiment of the present disclosure.

### DETAILED DESCRIPTION

The present disclosure provides a system and method for planning and performing microwave ablation surgical treatment. The system presents a clinician with a streamlined method of treatment planning from the initial patient selection through a process of target identification and selection, target sizing, treatment zone sizing, entry point and route selection to create a pathway to the target, and treatment plan review. The treatment plan may then be used as a guide during the performance of the surgical procedure, where the system is configured to track the position of surgical tools inside the patient and give the clinician a real-time view of the position of the tools in relation to the target and the pre-planned pathway toward the target. The system also presents a clinician with the capability to compare and contrast pre-operative and post-operative CT image data to assess the outcome of a surgical treatment procedure that has been performed.

Although the present disclosure will be described in terms of specific illustrative embodiments, it will be readily apparent to those skilled in this art that various modifications, rearrangements and substitutions may be made without departing from the present disclosure. The scope of the present disclosure is defined by the claims appended hereto.

Microwave ablation treatment, according to the present disclosure, is generally divided into two phases: (1) a planning phase, and (2) a procedure phase. The planning phase of microwave ablation treatment is more fully described in co-pending provisional patent application number 62/035,851 entitled TREATMENT PROCEDURE PLANNING SYSTEM AND METHOD, filed on August 11, 2014 by Bharadwaj et al. The alternative planning and a procedure phase are more fully described below.

A microwave ablation planning and procedure system according to the present disclosure may be a unitary system configured to perform both the planning phase and the procedure phase, or the system may include separate devices and software programs for the various phases. An example of the latter may be a system wherein a first computing device with one or more specialized software programs is used during the planning phase, and a second computing device with one or more specialized software programs may import data from the first computing device to be used during the procedure phase.

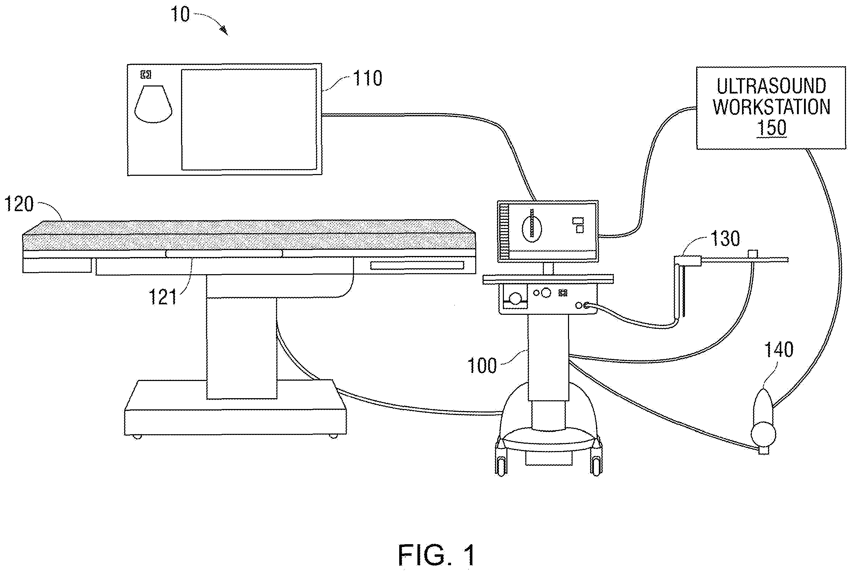

Referring now to FIG. 1, the present disclosure is generally directed to a treatment system 10, which includes a computing device 100, a display 110, a table 120, an ablation probe 130, and an ultrasound sensor 140. Computing device 100 may be, for example, a laptop computer, desktop computer, tablet computer, or other similar device. Computing device 100 may be configured to control an electrosurgical generator, a peristaltic pump, a power supply, and/or any other accessories and peripheral devices relating to, or forming part of, system 10. Display 110 is configured to output instructions, images, and messages relating to the performance of the microwave ablation procedure. Table 120 may be, for example, an operating table or other table suitable for use during a surgical procedure, which includes an electromagnetic (EM) field generator 121. EM field generator 121 is used to generate an EM field during the microwave ablation procedure and forms part of an EM tracking system which is used to track the positions of surgical instruments within the body of a patient. EM field generator 121 may include various components, such as a specially designed pad to be placed under, or integrated into, an operating table or patient bed. An example of such an EM tracking system is the AURORA^{™} system sold by Northern Digital Inc. Ablation probe 130 is a surgical instrument having a microwave ablation antenna which is used to ablate tissue. While the present disclosure describes the use of system 10 in a surgical environment, it is also envisioned that some or all of the components of system 10 may be used in alternative settings, for example, an imaging laboratory and/or an office setting.

In addition to the EM tracking system, the surgical instruments may also be visualized by using ultrasound imaging. Ultrasound sensor 140, such as an ultrasound wand, may be used to image the patient's body during the microwave ablation procedure to visualize the location of the surgical instruments, such as ablation probe 130, inside the patient's body. Ultrasound sensor 140 may have an EM tracking sensor embedded within or attached to the ultrasound wand, for example, a clip-on sensor or a sticker sensor. As described further below, ultrasound sensor 140 may be positioned in relation to ablation probe 130 such that ablation probe 130 is at an angle to the ultrasound image plane, thereby enabling the clinician to visualize the spatial relationship of ablation probe 130 with the ultrasound image plane and with objects being imaged. Further, the EM tracking system may also track the location of ultrasound sensor 140. In some embodiments, one or more ultrasound sensors 140 may be placed inside the body of the patient. EM tracking system may then track the location of such ultrasound sensors 140 and ablation probe 130 inside the body of the patient.

Various other surgical instruments or surgical tools, such as ligasure devices, surgical staples, etc., may also be used during the performance of a microwave ablation treatment procedure. Ablation probe 130 is used to ablate a lesion or tumor (hereinafter referred to as a "target") by using electromagnetic radiation or microwave energy to heat tissue in order to denature or kill cancerous cells. The construction and use of a system including such an ablation probe 130 is more fully described in co-pending provisional patent application no. 62/041,773 entitled MICROWAVE ABLATION SYSTEM, filed on August 26, 2014, by Dickhans, co-pending patent application no. 13/836,203 entitled MICROWAVE ABLATION CATHETER AND METHOD OF UTILIZING THE SAME, filed on March 15, 2013, by Latkow et al., and co-pending patent application no. 13/834,581 entitled MICROWAVE ENERGY-DELIVERY DEVICE AND SYSTEM, filed on March 15, 2013, by Brannan et al.

The location of ablation probe 130 within the body of the patient may be tracked during the surgical procedure. An example method of tracking the location of ablation probe 130 is by using the EM tracking system, which tracks the location of ablation probe 130 by tracking sensors attached to or incorporated in ablation probe 130. Various types of sensors may be used, such as a printed sensor, the construction and use of which is more fully described in co-pending provision patent application no. 62/095,563 filed December 22, 2014. Prior to starting the procedure, the clinician is able to verify the accuracy of the tracking system.

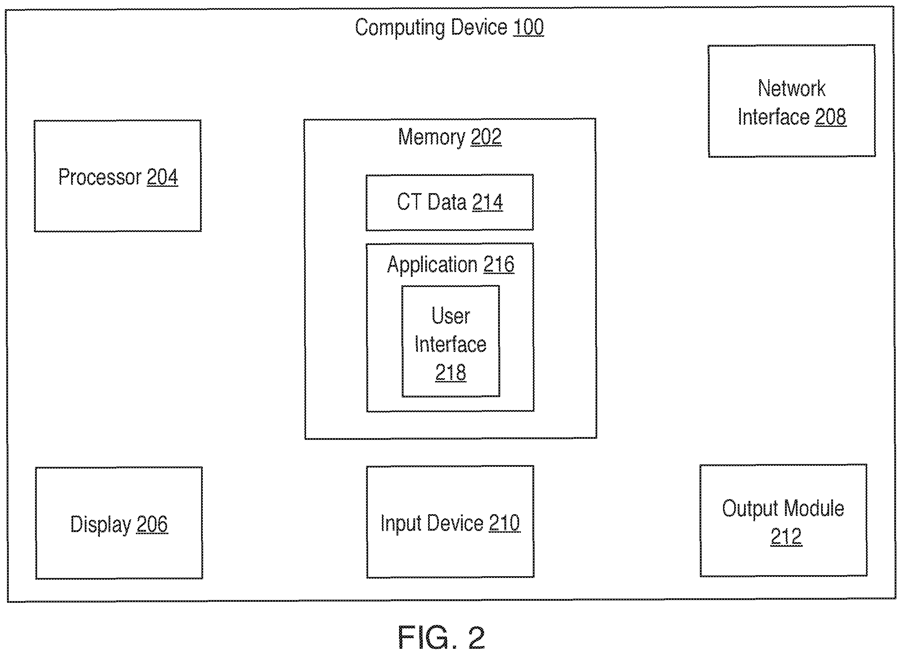

Turning now to FIG. 2, there is shown a system diagram of computing device 100. Computing device 100 may include memory 202, processor 204, display 206, network interface 208, input device 210, and/or output module 212.

Memory 202 includes any non-transitory computer-readable storage media for storing data and/or software that is executable by processor 204 and which controls the operation of computing device 100. In an embodiment, memory 202 may include one or more solid-state storage devices such as flash memory chips. Alternatively or in addition to the one or more solid-state storage devices, memory 202 may include one or more mass storage devices connected to the processor 204 through a mass storage controller (not shown) and a communications bus (not shown). Although the description of computer-readable media contained herein refers to a solid-state storage, it should be appreciated by those skilled in the art that computer-readable storage media can be any available media that can be accessed by the processor 204. That is, computer readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium which can be used to store the desired information and which can be accessed by computing device 100.

Memory 202 may store application 216 and/or CT data 214. Application 216 may, when executed by processor 204, cause display 206 to present user interface 218.

Processor 204 may be a general purpose processor, a specialized graphics processing unit (GPU) configured to perform specific graphics processing tasks while freeing up the general purpose processor to perform other tasks, and/or any number or combination of such processors.

Display 206 may be touch sensitive and/or voice activated, enabling display 206 to serve as both an input and output device. Alternatively, a keyboard (not shown), mouse (not shown), or other data input devices may be employed.

Network interface 208 may be configured to connect to a network such as a local area network (LAN) consisting of a wired network and/or a wireless network, a wide area network (WAN), a wireless mobile network, a Bluetooth network, and/or the internet. For example, computing device 100 may receive computed tomographic (CT) image data of a patient from a server, for example, a hospital server, internet server, or other similar servers, for use during surgical ablation planning. Patient CT image data may also be provided to computing device 100 via a removable memory 202. Computing device 100 may receive updates to its software, for example, application 216, via network interface 208. Computing device 100 may also display notifications on display 206 that a software update is available.

Input device 210 may be any device by means of which a user may interact with computing device 100, such as, for example, a mouse, keyboard, foot pedal, touch screen, and/or voice interface.

Output module 212 may include any connectivity port or bus, such as, for example, parallel ports, serial ports, universal serial busses (USB), or any other similar connectivity port known to those skilled in the art.

Application 216 may be one or more software programs stored in memory 202 and executed by processor 204 of computing device 100. As will be described in more detail below, during the planning phase, application 216 guides a clinician through a series of steps to identify a target, size the target, size a treatment zone, and/or determine an access route to the target for later use during the procedure phase. In some embodiments, application 216 is loaded on computing devices in an operating room or other facility where surgical procedures are performed, and is used as a plan or map to guide a clinician performing a surgical procedure, but without any feedback from ablation probe 130 used in the procedure to indicate where ablation probe 130 is located in relation to the plan. In other embodiments, system 10 provides computing device 100 with data regarding the location of ablation probe 130 within the body of the patient, such as by EM tracking, which application 216 may then use to indicate on the plan where ablation probe 130 are located.

Application 216 may be installed directly on computing device 100, or may be installed on another computer, for example a central server, and opened on computing device 100 via network interface 208. Application 216 may run natively on computing device 100, as a web-based application, or any other format known to those skilled in the art. In some embodiments, application 216 will be a single software program having all of the features and functionality described in the present disclosure. In other embodiments, application 216 may be two or more distinct software programs providing various parts of these features and functionality. For example, application 216 may include one software program for use during the planning phase, and a second software program for use during the procedure phase of the microwave ablation treatment. In such instances, the various software programs forming part of application 216 may be enabled to communicate with each other and/or import and export various settings and parameters relating to the microwave ablation treatment and/or the patient to share information. For example, a treatment plan and any of its components generated by one software program during the planning phase may be stored and exported to be used by a second software program during the procedure phase.

Application 216 communicates with a user interface 218 which generates a user interface for presenting visual interactive features to a clinician, for example, on display 206 and for receiving clinician input, for example, via a user input device. For example, user interface 218 may generate a graphical user interface (GUI) and output the GUI to display 206 for viewing by a clinician.

Computing device 100 is linked to display 110, thus enabling computing device 100 to control the output on display 110 along with the output on display 206. Computing device 100 may control display 110 to display output which is the same as or similar to the output displayed on display 206. For example, the output on display 206 may be mirrored on display 100. Alternatively, computing device 100 may control display 110 to display different output from that displayed on display 206. For example, display 110 may be controlled to display guidance images and information during the microwave ablation procedure, while display 206 is controlled to display other output, such as configuration or status information.

As used herein, the term "clinician" refers to any medical professional (i.e., doctor, surgeon, nurse, or the like) or other user of the treatment planning system 10 involved in planning, performing, monitoring and/or supervising a medical procedure involving the use of the embodiments described herein.

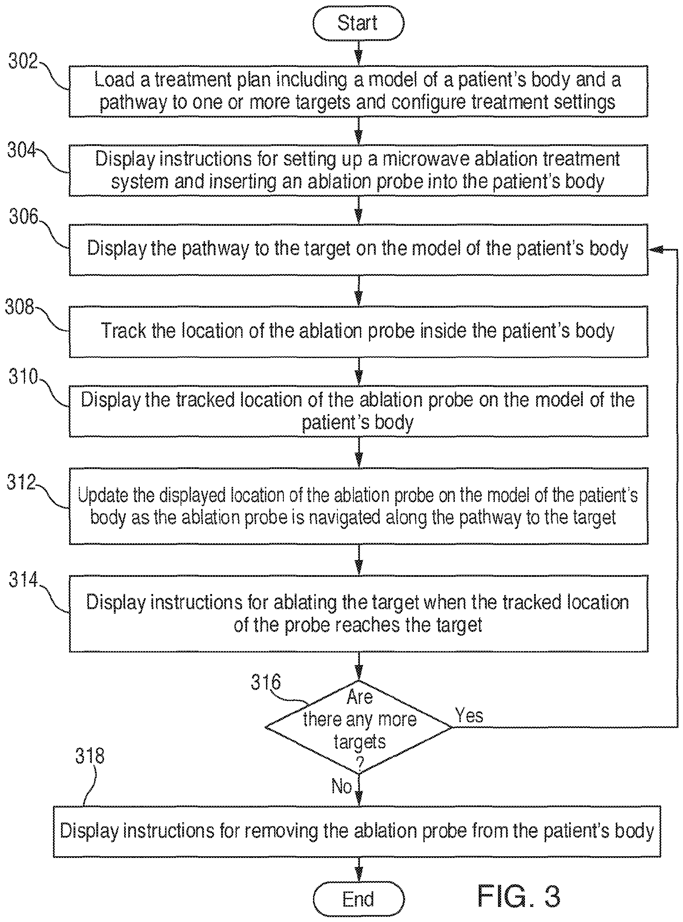

Turning now to Fig. 3, there is shown a flowchart of an example method for performing a microwave ablation procedure according to an embodiment of the present disclosure. At step 302, a clinician may use computing device 100 to load a treatment plan into application 216. The treatment plan may include a model of a patient's body and a pathway to one or more targets.

The model and treatment plan are both generated during the planning phase. The model may be generated based on CT image data acquired during a CT scan of the patient, although other imaging modalities are also envisioned. The clinician uses the model to select one or more targets for treatment during the microwave ablation procedure. Thereafter, application 216 generates a pathway from each selected target to an entry point on the patient's body where an ablation probe 130 may be inserted. The pathway is generated in such a way as to avoid any bones, vital organs, or other critical structures inside the patient's body. After loading the treatment plan on computing device 100, the clinician may view and modify the treatment plan.

The clinician may further configure the system settings for the microwave ablation procedure. For example, the clinician may preconfigure parameters related to the various tools to be used during the procedure, such as preconfiguring the output settings of ablation probe 130 for each target in the treatment plan. By doing so, application 216 may automatically configure a different output of ablation probe 130 when ablation probe 130 reaches each target.

The clinician may also view images or "snapshots" that were stored during the planning phase. For example, the clinician may store various images during the planning phase showing the targets from different angles. As noted above, the planning phase of microwave ablation treatment is more fully described in co-pending provisional patent application number 62/035,851 entitled TREATMENT PROCEDURE PLANNING SYSTEM AND METHOD.

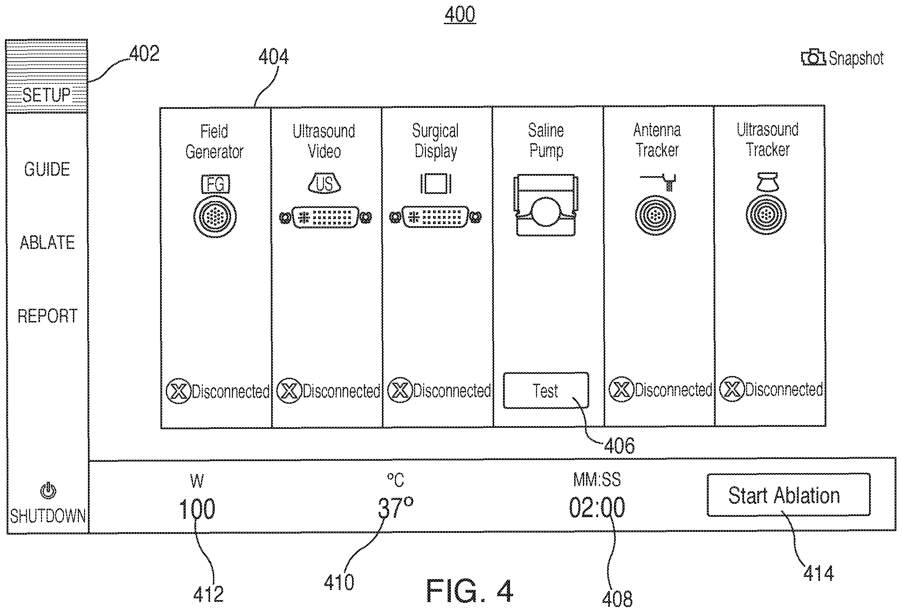

Then, at step 304, application 216, via user interface 218, displays instructions for setting up and configuring the microwave ablation system. The instructions may be visual and/or audible, and may provide feedback for proper versus improper system configuration. For example, as shown in Fig. 4, computing device 100 may display a system configuration screen 400. Screen 400 shows an indicator 402 of the step of ablation procedure in which the system is currently operating. Screen 400 further shows a list 404 which indicates various system components which should be connected for the procedure, as well as the status of those components. A button 406 is provided when a system component is connected to test the functioning of that component. Screen 400 also shows indicators representing the configured time 408, temperature 410, and output power 412 of ablation probe 130.

When the system has been configured for the procedure, the clinician may start the procedure, stop the procedure, pause the procedure, resume the procedure, and/or reset the procedure by selecting a button 414. Upon selecting button 414, application 216 causes computing device 100 to automatically start one or more of the system components. For example, application 216 may automatically start a peristaltic pump, an electrosurgical generator, and/or a power supply. Then, application 216 displays instructions for inserting ablation probe 130 into the patient's body. Thereafter, at step 306, application 216 displays the model of the patient's body with the pathway to the target as was generated in the planning phase.

In one embodiment, the treatment phase is similar to that employed by the iLogic^{®} system currently sold by Covidien LP, in which the position of the patient in the magnetic field is registered with the images from the planning phase. In addition the location of the ablation probe in the electromagnetic field is detected and displayed with reference to the planned pathway and the position of the patient and more specifically with respect to the target identified and displayed in the model.

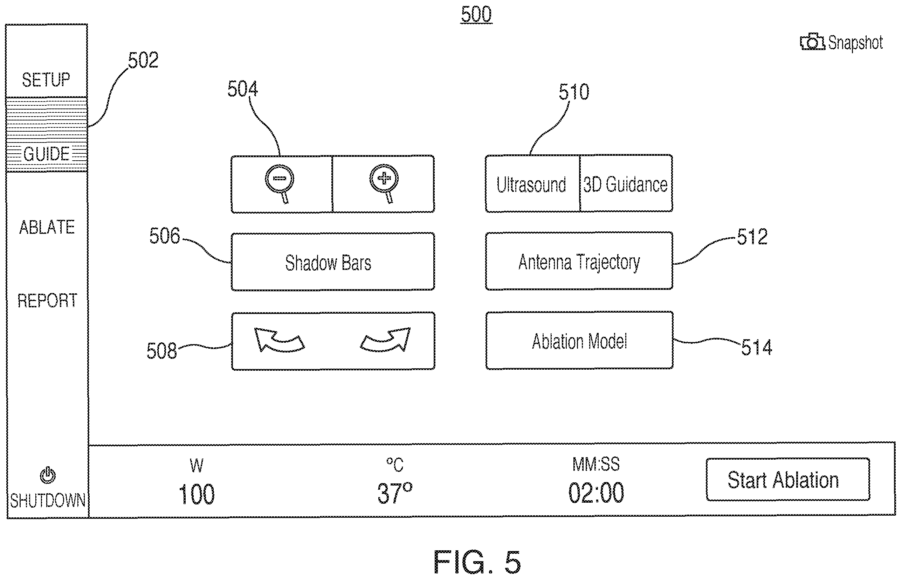

In an alternative or additional embodiment the clinician navigates ablation probe 130 along the pathway to the target utilizing the ultrasound imaging system including ultrasound sensor 140 and ultrasound workstation 150. The navigation instructions, such as the pathway and other relevant information, may be displayed on display 110, while display 206 displays a configuration screen 500, as shown in Fig. 5, described below. While ablation probe 130 is navigated, application 216, at step 308, tracks the location of ablation probe 130 inside the patient's body, and, at step 310, displays the tracked location of ablation probe 130 on the model of the patient's body. In addition, the application 216 projects a vector extending from the end of the ablation probe 130 to give an indication to the clinician of the intersecting tissue along the trajectory of the ablation probe 130. In this manner the clinician can alter the approach to a lesion or tumor to optimize the placement with a minimum of trauma.

Application 216, at step 312, iteratively updates the displayed location of ablation probe 130 on the model of the patient's body as ablation probe 130 is navigated along the pathway to the target.

When application 216 or the clinician detects that ablation probe 130 has reached the target, application 216, at step 314, displays instructions for ablating the target, including the settings previously set by the clinician for ablating the tumor, and enables the clinician to select the "start ablation" button to treat the target. When the "start ablation" button is selected, system 10 may automatically start other related accessories and/or peripheral devices, such as an associated peristaltic pump. Thereafter, at step 316, application 216 determines if there are any more targets in the treatment plan that have yet to be treated based on the planned procedure. If the determination is yes, the process returns to step 306 where the displayed pathway is updated to reflect the pathway to the next target. If the determination is no, application 216, at step 318, displays instructions for removing ablation probe 130 from the patient's body. During the ablation procedure, data relating to power and time settings as well as temperature data of ablation probe 130 for each ablation is continually stored.

Additionally, application 216 may present the clinician with instructions, such as a workflow, relating to protocols associated with a particular type of ablation procedure. For example, application 216 may present different workflows depending on the type of ablation procedure being performed, such that each of a track ablation, large tumor ablation, ablation of multiple tumors along a single track, and/or any other relevant ablation procedure may have instructions particularly tailored to the procedure.

Referring now to Fig. 5, there is shown an example screen 500 which may be displayed on display 206 either during the guidance step of the microwave ablation procedure or selected at any time by the clinician to adjust the features of the system 500. Screen 500 shows an indicator 502 that the system is now operating in the guidance step. Screen 500 further provides buttons 504 allowing the clinician to zoom in and out on the model and pathway displayed on display 110. Screen 500 further provides a button 506 which enables a shadow bar overlay on the pathway displayed on display 110 which indicates whether the trajectory of ablation probe 130 is in front of or behind an ultrasound image plane within the guidance view displayed on display 110. This enables the clinician to visualize the projected trajectory of ablation probe 130, as well as the interaction of the trajectory of ablation probe 130 within, or related to, the ultrasound image plane.

Screen 500 also includes buttons 508 allowing the clinician to rotate the guidance view displayed on display 110. Screen 500 further includes a button 510 allowing the clinician to toggle between a view of the model with the pathway and a live ultrasound image video feed. Screen 500 also includes a button 512 allowing the clinician to toggle the display of the planned pathway of ablation probe 130 on the model, and a button 514 allowing the clinician to toggle the display of a projected ablation zone relative to ablation probe 130 on the model to enable the clinician to visualize the ablation zone relative to ablation probe 130. The ablation zone may also be overlaid on the ultrasound images, thereby allowing the clinician to visualize the ablation zone within the ultrasound plane. The ablation zone may be presented to the clinician in a 2D and 3D ablation zone model.

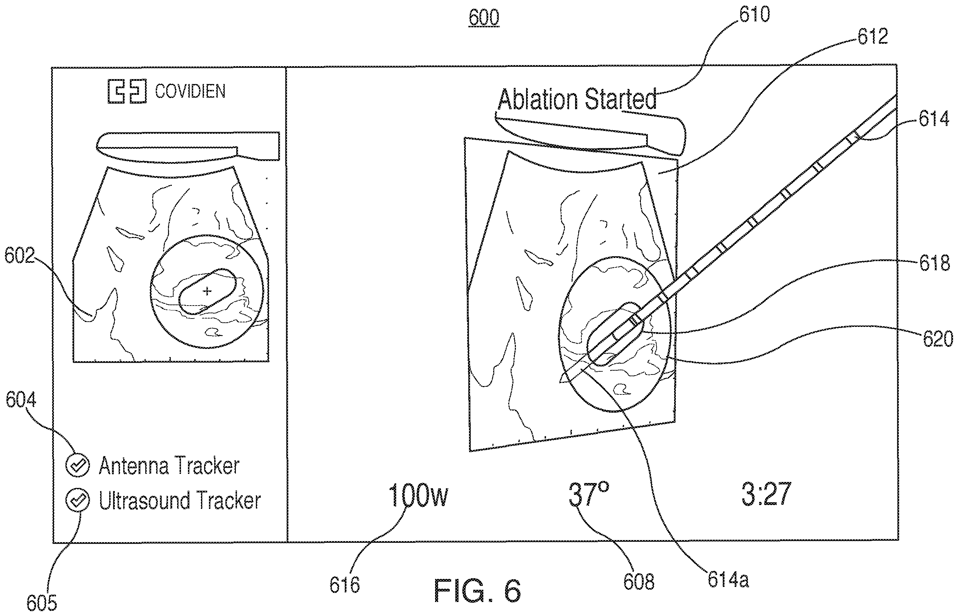

Fig. 6 shows an example screen 600 which may be displayed on display 110 during the microwave ablation procedure. Screen 600 includes a view 602 of the live 2D ultrasound images captured during the procedure. Screen 600 further shows a status indicator 604 for ablation probe 130 and a status indicator 606 for ultrasound sensor 140. Screen 600 also includes a view 608 for displaying status messages relating to the ablation procedure, such as a power setting of ablation probe 130, duration of the ablation and/or a time remaining until the ablation procedure is complete, progression of the ablation, feedback from a temperature sensor, and a zone chart used during the ablation procedure. Screen 600 further includes a view 610 for showing transient messages relating to the ablation procedure, such as changes caused by selecting the buttons provided by screen 500, described above. Screen 600 also displays the navigation view 612, which includes a representation 614 of ablation probe 130 as well as a shadow indicator 614a representing the portion of ablation probe 130 which lies below the ultrasound imaging plane, a vector line 616 representing the trajectory of the ablation probe, a current ablation zone 618 showing the area which is currently being ablated, and a total ablation zone 620 showing the area which will be ablated if the ablation procedure is allowed to run to completion.

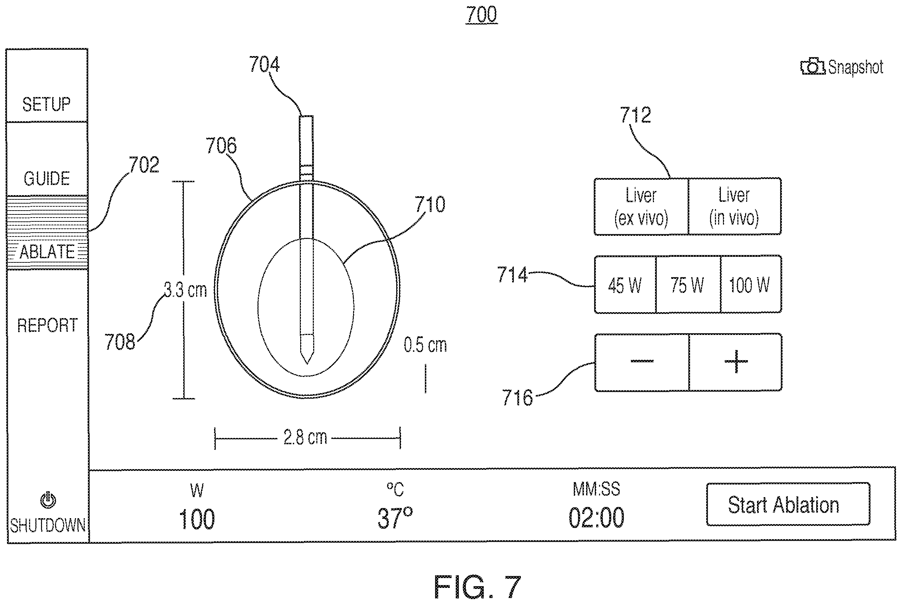

Fig. 7 shows an example screen 700 which may be displayed on display 206 during the ablation step of the microwave ablation procedure. Screen 700 shows an indicator 702 that the system is now operating in the ablation step. Screen 700 further shows a representation 704 of the surgical tool currently being used during the procedure, in the example ablation probe 130, and the ablation zone 706 based on the configured power and size of ablation probe 130, as well as the dimensions 708 of the ablation zone and a distance from the distal end of ablation probe 130 to the edge of the ablation zone. Screen 700 also shows a progress indicator 710 representing the progress of the ongoing ablation relative to ablation probe 130 and projected ablation zone 706. Screen 700 further includes a button 712 allowing the clinician to select a desired ablation zone chart based on the anatomical location of ablation probe 130 and in vivo or ex vivo data. Ex vivo data includes data acquired during an open surgical procedure, while in vivo data includes data acquired during other surgical procedures, such as laparoscopic surgical procedures. Screen 700 also includes a button 714 allowing the clinician to select a power setting for ablation probe 130, and a button 716 allowing the clinician to increase or decrease the size of the ablation zone based on the selected ablation zone chart.

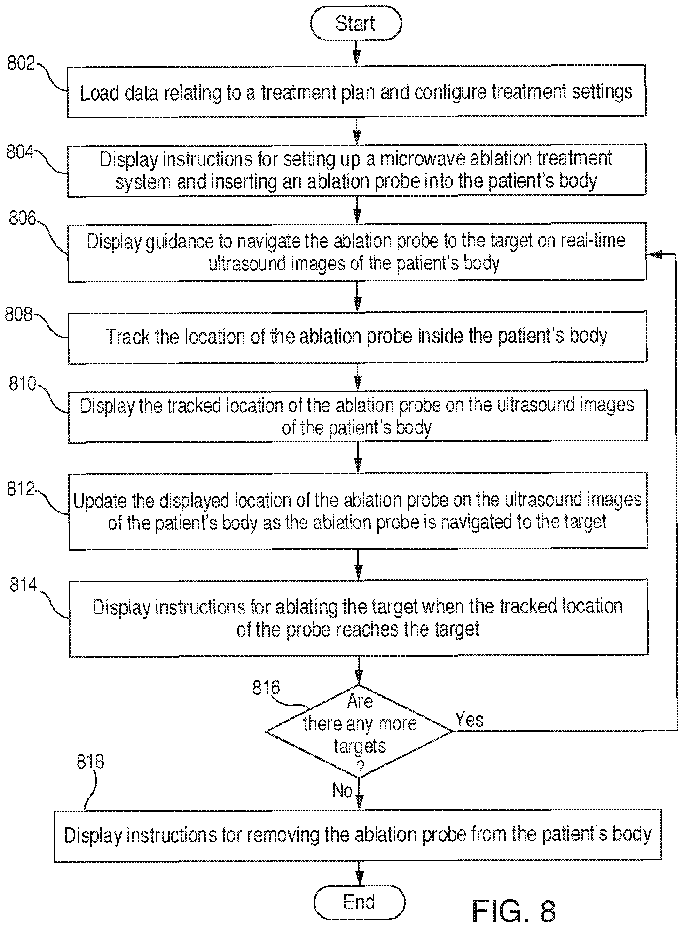

In some embodiments, system 10 may be operated without using the model generated during the planning phase of the microwave ablation treatment. In such embodiments, navigation of ablation probe 130 is guided by using ultrasound images, such as the ultrasound images generated by ultrasound sensor 140. During the guidance step of the microwave ablation procedure, the location of ablation probe 130 and the one or more targets are overlaid onto the ultrasound images generated by ultrasound sensor 140. By doing so, the location of ablation probe 130 may be viewed in relation to the ultrasound image plane to visualize a trajectory of ablation probe 130. The location of ablation probe 130 may be tracked by the EM tracking system, while the location of the one or more targets are determined based on data generated during the planning phase. A vector may also be displayed from the tip of ablation probe 130, showing the trajectory of ablation probe 130 and allowing the clinician to align ablation probe 130 to the target. An example method of performing a microwave ablation treatment procedure according to this embodiment is described below with reference to Fig. 8.

Referring now to Fig. 8, there is shown a flowchart of an example method for performing a microwave ablation procedure according to an embodiment of the present disclosure. At step 802, a clinician may use computing device 100 to load data relating to a treatment plan into application 216. The data may include the location of one or more targets within a patient's body, and a pathway to the one or more targets. The clinician may also configure the system settings for the microwave ablation procedure. For example, the clinician may preconfigure parameters related to the various tools to be used during the procedure, such as preconfiguring the output settings of ablation probe 130, such as a wattage, temperature, and/or duration of the ablation, for each target. By doing so, application 216 may automatically configure a different output of ablation probe 130 when ablation probe 130 reaches each target.

Then, at step 804, application 216, via user interface 218, displays instructions for setting up and configuring the microwave ablation system. Application 216 may also display instructions for inserting ablation probe 130 into the patient's body. Thereafter, at step 806, application 216 displays guidance to navigate ablation probe 130 to the target on ultrasound images generated by ultrasound sensor 140. The displayed guidance may include instructions for navigating ablation probe 130 to the one or more targets and/or a graphical map or pathway to the one or more targets which may be overlaid onto the ultrasound images.

The clinician then navigates ablation probe 130 to the target. While ablation probe 130 is navigated, application 216, at step 808, tracks the location of ablation probe 130 inside the patient's body, and, at step 810, displays the tracked location of ablation probe 130 on the ultrasound images of the patient's body generated by ultrasound sensor 140. Application 216, at step 812, iteratively updates the displayed location of ablation probe 130 on the ultrasound images as ablation probe 130 is navigated to the target.

When application 216 detects that ablation probe 130 has reached the target, application 216, at step 814, displays instructions for ablating the target. Thereafter, at step 816, application 216 determines if there are any more targets in the treatment plan that have yet to be treated. If the determination is yes, the process returns to step 806 where the guidance is updated to guide ablation probe 130 to the next target. If the determination is no, application 216, at step 818, displays instructions for removing ablation probe 130 from the patient's body.

In other embodiments, computing device 100 may be operated independently to control an electrosurgical generator. For example, as shown in Fig. 7, computing device 100 may display a control screen which enables a clinician to control an electrosurgical generator without interacting directly with the electrosurgical generator. The clinician may use computing device 100 to configure settings for the microwave ablation procedure. For example, the clinician may preconfigure output wattages and ablation zones for each target to be ablated during the procedure, as well as other settings related to the operation of the electrosurgical generator during the procedure.

## Claims

1. A system for performing a microwave ablation procedure, the system comprising:
an ablation probe (130);
an electromagnetic tracking system configured to track the location of the ablation probe inside a patient's body by using at least one electromagnetic sensor located on the ablation probe while the ablation probe is navigated inside the patient's body;
an ultrasound imager (140) configured to generate real-time ultrasound images; and
a computing device (100) including a processor (204) and a memory (202) storing instructions which, when executed by the processor, cause the computing device to:
display guidance for navigating the ablation probe to at least one ablation target within the patient's body;
display the tracked location of the ablation probe on the real-time ultrasound images based on the tracked location of the ablation probe inside the patient's body, wherein the location of the ablation probe is displayed in relation to the at least one target;
iteratively update the displayed location of the ablation probe as the location of the ablation probe is tracked while the ablation probe is navigated inside the patient's body;
display guidance for ablating the at least one target when the ablation probe is navigated proximate to the at least one target;
display a model of a planned ablation zone (620) in relation to the at least one target on the real-time ultrasound images; and characterized to further
display a current ablation zone (618) showing an area which is currently being ablated together with the model of the planned ablation zone on the real-time ultrasound images.

2. The system according to claim 1, wherein the electromagnetic tracking system tracks the location of the ultrasound imager by using at least one electromagnetic sensor located on the ultrasound imager.

3. The system according to claim 2, wherein the location of the ablation probe in relation to the at least one target is displayed on the real-time ultrasound images based on the tracked location of the ultrasound imager.

4. The system according to claim 1, wherein the location of the ablation probe in relation to the at least one target is displayed on the real-time ultrasound images based on the tracked location of the ablation probe inside the patient's body.

5. The system according to claim 1, wherein the displayed location of the ablation probe is iteratively updated in relation to the at least one target.

6. The system according to any preceding claim, further comprising one or more peripheral components which are automatically started by the computing device, wherein the one or more peripheral components include one or more of an ablation generator, a peristaltic pump, and a smart power supply.

7. The system according to any preceding claim, wherein the instructions further configure the computing device to display, on the real-time ultrasound images, a vector (616) from the tip of the ablation probe indicating the trajectory of the ablation probe, preferably wherein the instructions further configure the computing device to display, on the real-time ultrasound images, a shadow bar overlay (614a) indicating whether the trajectory of the ablation probe is in front of or behind the plane of the real-time ultrasound images.

8. The system according to claim 1, wherein the computing device enables a user to preconfigure at least one parameter regarding ablating the at least one target, preferably wherein the at least one parameter includes one or more of a wattage, temperature, and duration.

9. A non-transitory computer-readable storage medium storing instructions which, when executed by a processor, cause a computing device to:
display guidance for navigating an ablation probe to at least one ablation target within a patient's body;
track the location of the ablation probe inside the patient's body while the ablation probe is navigated;
display the tracked of the ablation probe on real-time ultrasound images, wherein the location of the ablation probe is displayed in relation to the at least one target;
iteratively update the displayed location of the ablation probe as the location of the ablation probe is tracked while the ablation probe is navigated inside the patient's body;
display guidance for ablating the at least one target when the ablation probe is navigated proximate to the at least one target;
and characterized to further
display a model of a planned ablation zone in relation to the at least one target together with a current ablation zone showing an area which is currently being ablated on the real-time ultrasound images.

10. The non-transitory computer-readable medium according to claim 9, comprising further instructions which configure the computing device to track the location of at least one electromagnetic sensor located on the ablation probe.

11. The non-transitory computer-readable medium according to claim 9, wherein the location of the ablation probe in relation to the at least one target is displayed on the real-time ultrasound images based on the tracked location of the ablation probe inside the patient's body.

## Patentansprüche

1. System zum Durchführen eines Mikrowellenablationsverfahrens, das System Folgendes umfassend:
eine Ablationssonde (130);
ein elektromagnetisches Verfolgungssystem, das eingerichtet ist, um den Ort der Ablationssonde im Körper eines Patienten unter Verwendung mindestens eines elektromagnetischen Sensors zu verfolgen, der sich auf der Ablationssonde befindet, während die Ablationssonde im Körper des Patienten navigiert wird;
einen Ultraschallbildgeber (140), der eingerichtet ist, um Echtzeit-Ultraschallbilder zu erzeugen; und
eine Datenverarbeitungsvorrichtung (100), die einen Prozessor (204) und einen Speicher (202) umfasst, der Befehle speichert, die, wenn sie von dem Prozessor ausgeführt werden, bewirken, dass die Datenverarbeitungsvorrichtung:
eine Führung zum Navigieren der Ablationssonde zu mindestens einem Ablationsziel im Körper des Patienten anzeigt;
den verfolgten Ort der Ablationssonde auf den Echtzeit-Ultraschallbildern auf Grundlage des verfolgten Ortes der Ablationssonde im Körper des Patienten anzeigt, wobei der Ort der Ablationssonde in Bezug auf das mindestens eine Ziel angezeigt wird;
den angezeigten Ort der Ablationssonde iterativ aktualisiert, wenn der Ort der Ablationssonde verfolgt wird, während die Ablationssonde im Körper des Patienten navigiert wird;
eine Führung zum Abladieren des mindestens einen Ziels anzeigt, wenn die Ablationssonde in die Nähe des mindestens einen Ziels navigiert wird;
ein Modell einer geplanten Ablationszone (620) in Bezug auf das mindestens eine Ziel auf den Echtzeit-Ultraschallbildern anzeigt;
und **dadurch gekennzeichnet, dass** sie weiterhin
eine aktuelle Ablationszone (618) anzeigt, die einen Bereich, der gegenwärtig abladiert wird, zusammen mit dem Modell der geplanten Ablationszone auf den Echtzeit-Ultraschallbildern zeigt.

2. System nach Anspruch 1, wobei das elektromagnetische Verfolgungssystem den Ort des Ultraschallbildgebers unter Verwendung mindestens eines elektromagnetischen Sensors verfolgt, der sich auf dem Ultraschallbildgeber befindet.

3. System nach Anspruch 2, wobei der Ort der Ablationssonde in Bezug auf das mindestens eine Ziel auf den Echtzeit-Ultraschallbildern auf Grundlage des verfolgten Ortes des Ultraschallbildgebers angezeigt wird.

4. System nach Anspruch 1, wobei der Ort der Ablationssonde in Bezug auf das mindestens eine Ziel auf den Echtzeit-Ultraschallbildern auf Grundlage des verfolgten Orts der Ablationssonde im Körper des Patienten angezeigt wird.

5. System nach Anspruch 1, wobei der angezeigte Ort der Ablationssonde in Bezug auf das mindestens eine Ziel iterativ aktualisiert wird.

6. System nach einem der vorhergehenden Ansprüche, weiterhin mindestens eine Peripheriekomponente umfassend, die automatisch durch die Datenverarbeitungsvorrichtung gestartet wird, wobei die mindestens eine Peripheriekomponente mindestens eines von einem Ablationsgenerator, einer Peristaltikpumpe und einer intelligenten Stromversorgung umfasst.

7. System nach einem der vorhergehenden Ansprüche, wobei die Befehle die Datenverarbeitungsvorrichtung weiterhin so einrichten, dass sie auf den Echtzeit-Ultraschallbildern einen Vektor (616) von der Spitze der Ablationssonde anzeigt, der die Trajektorie der Ablationssonde anzeigt, wobei die Befehle vorzugsweise die Datenverarbeitungsvorrichtung weiterhin so einrichten, dass sie auf den Echtzeit-Ultraschallbildern eine Schattenbalkenüberlagerung (614a) anzeigt, die angibt, ob die Trajektorie der Ablationssonde vor oder hinter der Ebene der Echtzeit-Ultraschallbilder ist.

8. System nach Anspruch 1, wobei die Datenverarbeitungsvorrichtung einem Benutzer ermöglicht, mindestens einen Parameter hinsichtlich eines Abladierens des mindestens einen Ziels zuvor einzurichten, wobei der mindestens eine Parameter vorzugsweise mindestens einer von einer Wattleistung, Temperatur und Dauer umfasst.

9. Nichtflüchtiges computerlesbares Speichermedium, das Befehle speichert, die, wenn sie durch einen Prozessor ausgeführt werden, bewirken, dass eine Datenverarbeitungsvorrichtung:
eine Führung zum Navigieren einer Ablationssonde zu mindestens einem Ablationsziel im Körper eines Patienten anzeigt;
den Ort der Ablationssonde im Körper des Patienten verfolgt, während die Ablationssonde navigiert wird;
den verfolgten Ort der Ablationssonde auf Echtzeit-Ultraschallbildern anzeigt, wobei der Ort der Ablationssonde in Bezug auf das mindestens eine Ziel angezeigt wird;
den angezeigten Ort der Ablationssonde iterativ aktualisiert, wenn der Ort der Ablationssonde verfolgt wird, während die Ablationssonde im Körper des Patienten navigiert wird;
eine Führung zum Abladieren des mindestens einen Ziels anzeigt, wenn die Ablationssonde in die Nähe des mindestens einen Ziels navigiert wird;
und **dadurch gekennzeichnet, dass** sie weiterhin
ein Modell einer geplanten Ablationszone in Bezug auf das mindestens eine Ziel zusammen mit einer aktuellen Ablationszone anzeigt, die einen Bereich zeigt, der gegenwärtig auf den Echtzeit-Ultraschallbildern abladiert wird.

10. Nichtflüchtiges computerlesbares Medium nach Anspruch 9, das weiterhin Befehle umfasst, welche die Datenverarbeitungsvorrichtung einrichten, um den Ort mindestens eines elektromagnetischen Sensors zu verfolgen, der sich auf der Ablationssonde befindet.

11. Nichtflüchtiges computerlesbares Medium nach Anspruch 9, wobei der Ort der Ablationssonde in Bezug auf das mindestens eine Ziel auf den Echtzeit-Ultraschallbildern auf Grundlage des verfolgten Orts der Ablationssonde im Körper des Patienten angezeigt wird.

## Revendications

1. Système permettant d'effectuer une intervention d'ablation par micro-ondes, le système comprenant :
une sonde d'ablation (130) ;
un système de suivi électromagnétique configuré pour suivre l'emplacement de la sonde d'ablation à l'intérieur du corps d'un patient à l'aide d'au moins un capteur électromagnétique situé sur la sonde d'ablation pendant que l'on fait naviguer la sonde d'ablation à l'intérieur du corps du patient ;
un imageur ultrasonore (140) configuré pour générer des images ultrasonores en temps réel ; et
un dispositif informatique (100) comprenant un processeur (204) et une mémoire (202) stockant des instructions qui, lorsqu'elles sont exécutées par le processeur, amènent le dispositif informatique à :
afficher un guidage pour la navigation de la sonde d'ablation vers au moins une cible d'ablation à l'intérieur du corps du patient ;
afficher l'emplacement suivi de la sonde d'ablation sur les images ultrasonores en temps réel en fonction de l'emplacement suivi de la sonde d'ablation à l'intérieur du corps du patient, dans lequel l'emplacement de la sonde d'ablation est affiché par rapport à l'au moins une cible ;
mettre à jour de manière itérative l'emplacement affiché de la sonde d'ablation à mesure que l'emplacement de la sonde d'ablation est suivi pendant que l'on fait naviguer la sonde d'ablation à l'intérieur du corps du patient ;
afficher un guidage pour l'ablation de l'au moins une cible lorsque l'on fait naviguer la sonde d'ablation à proximité de l'au moins une cible ;
afficher un modèle d'une zone d'ablation planifiée (620) en relation avec l'au moins une cible sur les images ultrasonores en temps réel ; et caractérisé en outre pour
afficher une zone d'ablation actuelle (618) montrant une zone en cours d'ablation conjointement avec le modèle de la zone d'ablation planifiée sur les images ultrasonores en temps réel.

2. Système selon la revendication 1, dans lequel le système de suivi électromagnétique suit l'emplacement de l'imageur ultrasonore à l'aide d'au moins un capteur électromagnétique situé sur l'imageur ultrasonore.

3. Système selon la revendication 2, dans lequel l'emplacement de la sonde d'ablation par rapport à l'au moins une cible est affiché sur les images ultrasonores en temps réel en fonction de l'emplacement suivi de l'imageur ultrasonore.

4. Système selon la revendication 1, dans lequel l'emplacement de la sonde d'ablation par rapport à l'au moins une cible est affiché sur les images ultrasonores en temps réel sur la base de l'emplacement suivi de la sonde d'ablation à l'intérieur du corps du patient.

5. Système selon la revendication 1, dans lequel l'emplacement affiché de la sonde d'ablation est mis à jour de manière itérative par rapport à l'au moins une cible.

6. Système selon selon une quelconque revendication précédente, comprenant en outre un ou plusieurs composants périphériques qui sont automatiquement démarrés par le dispositif informatique, dans lequel les un ou plusieurs composants périphériques comprennent un ou plusieurs d'un générateur d'ablation, d'une pompe péristaltique et d'une alimentation électrique intelligente.

7. Système selon une quelconque revendication précédente, dans lequel les instructions configurent en outre le dispositif informatique pour afficher, sur les images ultrasonores en temps réel, un vecteur (616) à partir de la pointe de la sonde d'ablation indiquant la trajectoire de la sonde d'ablation, de préférence dans lequel les instructions configurent en outre le dispositif informatique pour afficher, sur les images ultrasonores en temps réel, une barre d'ombre superposée (614a) indiquant si la trajectoire de la sonde d'ablation est devant ou derrière le plan des images ultrasonores en temps réel.

8. Système selon la revendication 1, dans lequel le dispositif informatique permet à un utilisateur de préconfigurer au moins un paramètre concernant l'ablation de l'au moins une cible, de préférence dans lequel l'au moins un paramètre comprend une ou plusieurs d'une puissance, d'une température et d'une durée.

9. Support de stockage non transitoire lisible par ordinateur stockant des instructions qui, lorsqu'elles sont exécutées par un processeur, amènent un dispositif informatique à :
afficher un guidage pour la navigation d'une sonde d'ablation vers au moins une cible d'ablation à l'intérieur du corps d'un patient ;
suivre l'emplacement de la sonde d'ablation à l'intérieur du corps du patient pendant que l'on fait naviguer la sonde d'ablation ;
afficher le suivi de la sonde d'ablation sur des images ultrasonores en temps réel, dans lequel l'emplacement de la sonde d'ablation est affiché par rapport à l'au moins une cible ;
mettre à jour de manière itérative l'emplacement affiché de la sonde d'ablation à mesure que l'emplacement de la sonde d'ablation est suivi pendant que l'on fait naviguer la sonde d'ablation à l'intérieur du corps du patient ;
afficher un guidage pour l'ablation de l'au moins une cible lorsque l'on fait naviguer la sonde d'ablation à proximité de l'au moins une cible ;
et caractérisé pour en outre
afficher un modèle d'une zone d'ablation planifiée par rapport à l'au moins une cible conjointement avec une zone d'ablation actuelle montrant une surface en cours d'ablation sur les images ultrasonores en temps réel.

10. Support non transitoire lisible par ordinateur selon la revendication 9, comprenant d'autres instructions qui configurent le dispositif informatique pour suivre l'emplacement d'au moins un capteur électromagnétique situé sur la sonde d'ablation.

11. Support non transitoire lisible par ordinateur selon la revendication 9, dans lequel l'emplacement de la sonde d'ablation par rapport à l'au moins une cible est affiché sur les images ultrasonores en temps réel sur la base de l'emplacement suivi de la sonde d'ablation à l'intérieur du corps du patient.
